# EUROPEAN PATENT SPECIFICATION

(11) **EP 0 804 479 B1**
(45) Date of publication and mention of the grant of the patent: **11.10.2006**
(21) Application number: 95934808.7
(22) Date of filing: 12.10.1995
(51) Int. Cl.: C07K 14/50, A61K 38/18

(54) **METHOD OF TREATING DIABETES MELLITUS USING KGF**
VERFAHREN ZUR BEHANDLUNG VON DIABETES MITTELS KGF
PROCEDE DE TRAITEMENT DU DIABETE SUCRE A L'AIDE DU FACTEUR DE CROISSANCE DES KERATINOCYTES

(30) Priority: 13.10.1994 US 323340; 13.10.1994 US 323475; 07.06.1995 US 487825
(43) Date of publication of application: 05.11.1997
(73) Proprietor: Amgen Inc.,, Thousand Oaks, California 91320-1799 (US)
(72) Inventor: AUKERMAN, Sharon, Lea, Thousand Oaks, CA 91360 (US); PIERCE, Glenn, Francis, Rancho Santa Fe, CA 92067 (US)
(74) Representative: Grünecker, Kinkeldey, Stockmair & Schwanhäusser Anwaltssozietät
(86) International application number: PCT/IB1995/000992
(87) International publication number: WO 1996/011950

(56) References cited:
- EP-A- 0 303 233
- EP-A- 0 619 370
- BRITISH MED.BULL., vol. 45, no. 2, 1989 pages 438-452, A.BAIRD E A 'Fibroblast growth factors'

### SEQUENCE LISTING

(1) GENERAL INFORMATION:
   (i) APPLICANT: Amgen Inc.
   (ii) TITLE Of INVENTION: Method of Treating Diabetes Mellitus Using KGF
   (iii) NUMBER OF SEQUENCES: 2
   (iv) CORRESPONDENCE ADDRESS:
      (A) ADDRESSEE: Amgen Inc.
      (8) STREET: 1840 DeHavilland Drive
      (C) CITY: Thousand Oaks
      (D) STATE: California
      (B) COUNTRY: U.S.A.
      (F) ZIP: 91320-1789
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: US PCT/IB 95/00992
      (B) FILING DATE: 12-OCT-1995
      (C) CLASSIFICATION:
(2) INFORMATION FOR SEQ ID NO:1:
   (i) SEQUENCE CHARACTERISTICS:
      (A) LENGTH: 862 base pairs
      (B) TYPE: nucleic acid
      (C) STRANDEDNESS: unknown
      (D) TOPOLOGY: unknown
   (ii) MOLECULE TYPE: cDNA
   (xi) SEQUENCE DESCRIPTION: SEQ ID NO:1:
   CAATCTACAA TTCACAGATA GGAAGAGGTC AATGACCTAG GAGTAACAAT CAACTCAAGA    60
   TTCATTTTCA TTATGTTATT CATGAACACC CGGAGCACTA CACTATAATG CACAAATGGA   120
   TACTGACATG GATCCTGCCA ACTTTGCTCT ACAGATCATG CTTTCACATT ATCTGTCTAG   180
   TGGGTACTAT ATCTTTAGCT TGCAATGACA TGACTCCAGA GCAAATGGCT ACAAATGTGA   240
   ACTGTTCCAG CCCTGAGCGA CACACAAGAA GTTATGATTA CATGGAAGGA GGGGATATAA   300
   GAGTGAGAAG ACTCTTCTGT CGAACACAGT GGTACCTGAG GATCGATAAA AGAGGCAAAG   360
   TAAAAGGGAC CCAAGAGATG AAGAATAATT ACAATATCAT GGAAATCAGG ACAGTGGCAG   420
   TTGGAATTGT GGCAATCAAA GGGGTGGAAA GTGAATTCTA TCTTGCAATG AACAAGGAAG   480
   GAAAACTCTA TGCAAAGAAA GAATGCAATG AAGATTGTAA CTTCAAAGAA CTAATTCTGG   540
   AAAACCATTA CAACACATAT GCATCAGCTA AATGGACACA CAACGGAGGG GAAATGTTTG   600
   TTGCCTTAAA TCAAAAGGGG ATTCCTGTAA GAGGAAAAAA AACGAAGAAA GAACAAAAAA   660
   CAGCCCACTT TCTTCCTATG GCAATAACTT AATTGCATAT GGTATATAAA GAACCCAGTT   720
   CCAGCAGGGA GATTTCTTTA AGTGGACTGT TTTCTTTCTT CTCAAAATTT TCTTTCCTTT   780
   TATTTTTTAG TAATCAAGAA AGGCTGGAAA AACTACTGAA AAACTGATCA AGCTGGACTT   840
   GTGCATTTAT GTTTGTTTTA AG                                            862
(2) INFORMATION FOR SEQ ID NO:2:
   (i) SEQUENCE CRARACTERISTICS;
      (A) LENGTH: 194 amino acids
      (8) TYPE: amino acid
      (C) STRANDEDNESS: unknown
      (D) TOPOLOGY: unknown
   (ii) MOLECULE TYPE: peptide
   (xi) SEQUENCE DESCRIPTION: SEQ ID NO:2:
   Met His Lys Trp Ile Leu Thr Trp Ile Leu Pro Thr Leu Leu Tyr Arg
   1               5                   10                  15
   Ser Cys Phe His Ile Ile Cys Leu Val Gly Thr Ile Ser Leu Ala Cys
               20                  25                  30
   Asn Asp Met Thr Pro Glu Gln Met Ala Thr Asn Val Asn Cys Ser Ser
           35                  40                  45
   Pro Glu Arg His Thr Arg Ser Tyr Asp Tyr Met Glu Gly Gly Asp Ile
       50                  55                  60
   Arg Val Arg Arg Leu Phe Cys Arg Thr Gln Trp Tyr Leu Arg Ile Asp
   65                  70                  75                  80
   Lys Arg Gly Lys Val Lys Gly Thr Gln Glu Met Lys Asn Asn Tyr Asn
                   85                  90                  95
   Ile Met Glu Ile Arg Thr Val Ala Val Gly Ile Val Ala Ile Lys Gly
               100                 105                 110
   Val Glu Ser Glu Phe Tyr Leu Ala Met Asn Lys Glu Gly Lys Leu Tyr
           115                 120                 125
   Ala Lys Lys Glu Cys Asn Glu Asp Cys Asn Phe Lys Glu Leu Ile Leu
       130                 135                 140
   Glu Asn His Tyr Asn Thr Tyr Ala Ser Ala Lys Trp Thr His Asn Gly
   145                 150                 155                 160
   Gly Glu Met Phe Val Ala Leu Asn Gln Lys Gly Ile Pro Val Arg Gly
                   165                 170                 175
   Lys Lys Thr Lys Lys Glu Gln Lys Thr Ala His Phe Leu Pro Met Ala
               180                 185                 190
   Ile Thr

## Description

### FIELD OF THE INVENTION

The present invention relates to the application of keratinocyte growth factor for preparing a medicament to treat or prevent the onset of diabetes mellitus.

### BACKGROUND OF THE INVENTION

Keratinocyte growth factor (KGF) is a growth factor specific for epithelial cells that was first identified in conditioned medium of a human embryonic lung fibroblast cell line. Rubin et al., *Proc. Natl. Acad. Sci. USA* **86**:802-806 (1989). Expression of messenger RNA for KGF has been detected in several stromal fibroblast cell lines derived from epithelial tissues at various stages of development. The transcript for KGF was also evident in RNA extracted from normal adult kidney and organs of the gastrointestinal tract. Finch et al., Science **245**:752-755 (1989). Evidence that KGF is secreted from fibroblasts in culture and is expressed *in vivo* in the dermis but not epidermis indicates that KGF may be an important normal paracrine effector of keratinocyte proliferation. Studies have shown that KGF is as potent as EGF in stimulating the proliferation of primary or secondary human keratinocytes in tissue culture. Marchese et al., *J. Cell. Phys.* **144**:326-332 (1990).

*Ex vivo* and *in vivo* studies in normal adult animals have shown that KGF produces changes in hair follicle morphogenesis, hepatocyte proliferation, and epithelial cell proliferation in the lung, breast, pancreas, stomach, small intestine, and large intestine. Panos et *al., J. Clin. Invest.* **92:**969-977 (1993); Ulich *et al., Am. J. Path.* **144:**862-868 (1994); Yi et *al., Am. J. Path.* **145:**80-85 (1994); and Ulich *et al., J. Clin. Invest.* **93:**1298-1306 (1994). The role of KGF in embryonic or neonatal development has not been studied in detail; however, KGF has been documented to be an important mediator of seminal vesicle development in the newborn mouse. Alarid *et al., P.N.A.S.* **91:**1074-1078 (1994).

Published PCT patent application WO 90/08771 describes the purification of KGF from the conditioned medium of a human embryonic fibroblast cell line, the partial amino acid sequencing of purified KGF, the cloning of the gene, and the expression of the gene in bacterial cells to yield biologically active recombinant KGF. The aforementioned publication discloses that KGF or KGF-like polypeptides can be used as wound healing agents for burn wounds or to stimulate transplanted corneal tissue. In fact, KGF has been demonstrated to increase re-epithelialization and increased thickness of the epithelium when recombinant KGF was topically applied to wounds surgically induced in the rabbit ear or in porcine skin. Pierce *et al., J. Exp. Med.*, **179:**831-840 (1994); and Staiano-Coico *et al., J. Exp. Med.* **178:**865-878 (1993).

EP 0303233 discloses human reg protein which is capable of regenerating insulin-producing pancreas islet B cells. British Med. Bull., Vol. 45, (1989), p. 438-452 is a review about Fibroblast Growth Factors (FGFs) which among others discloses the role of FGFs in diabetes.

### SUMMARY OF THE INVENTION

The discovery has now been made that KGF is useful to treat the medical disorder known as diabetes.

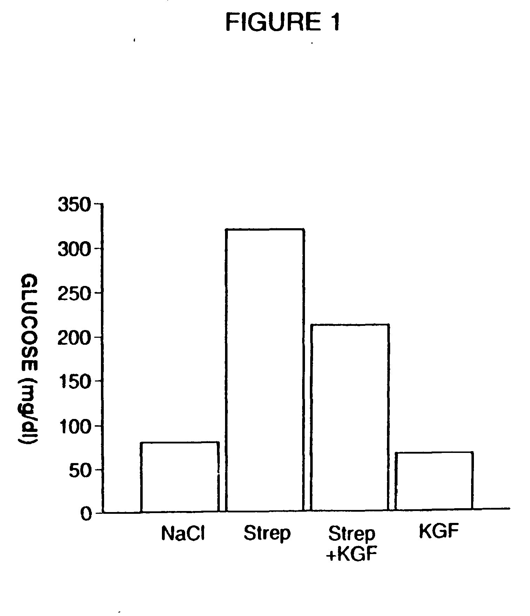

Figure 1 is a bar graph depicting the effects of KGF in rats following daily subcutaneous administration at a dose of 5 milligrams per kilogram of body weight (mg/kg) over seven days. Streptozotocin (55 mg/kg) was administered once intravenously two days after the initiation of KGF treatment. The KGF-treated group of diabetic rats is shown in the right half of the figure, above the legend "Strep + KGF". Control groups are represented to the left and right of that: treatment over seven days with sodium chloride solution and no diabetes induction ("NaC1"), treatment over seven days with sodium chloride solution before and after streptozotocin-induced diabetes ("Strep"), and treatment over seven days with KGF and no diabetes induction ("KGF"). Non-fasting blood glucose levels in milligrams per deciliter (mg/dl) are shown on the vertical axis, as measured on the fifth day after diabetes induction (i.e., seventh day after KGF or sodium chloride treatment was initiated). There were four rats per group.

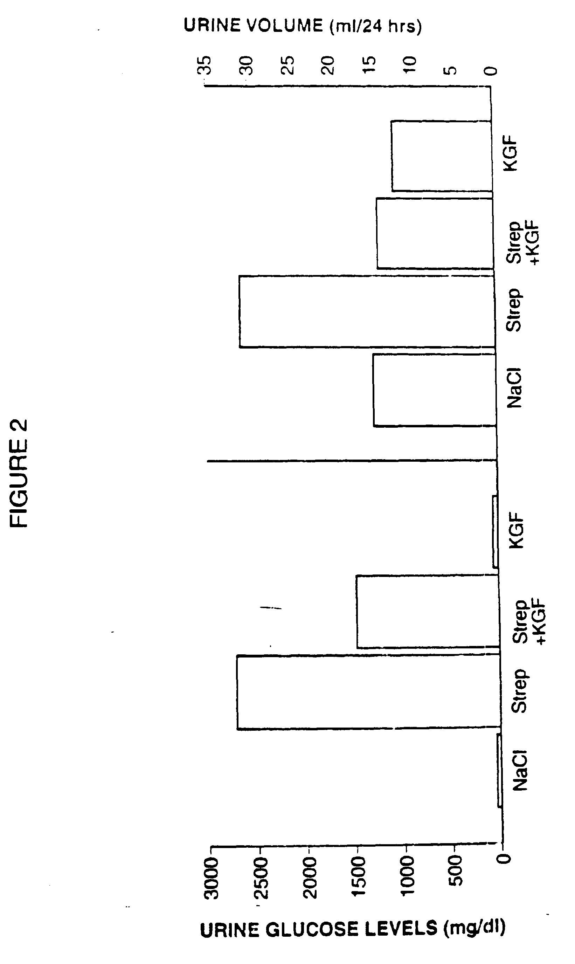

Figure 2 is a bar graph depicting the effect of KGF in the same rat model on other physiological measurements relating to diabetes. Fasting urine glucose levels in mg/dl and fasting urine output in milliliter (ml) excreted in twenty four hours on the seventh day of KGF or sodium chloride treatment are shown on the vertical axis, left half and right half, respectively. Legends ("NaCl", "Strep", "Strep + KGF" and "KGF") have the same meanings as in Figure 1. There were four rats per group.

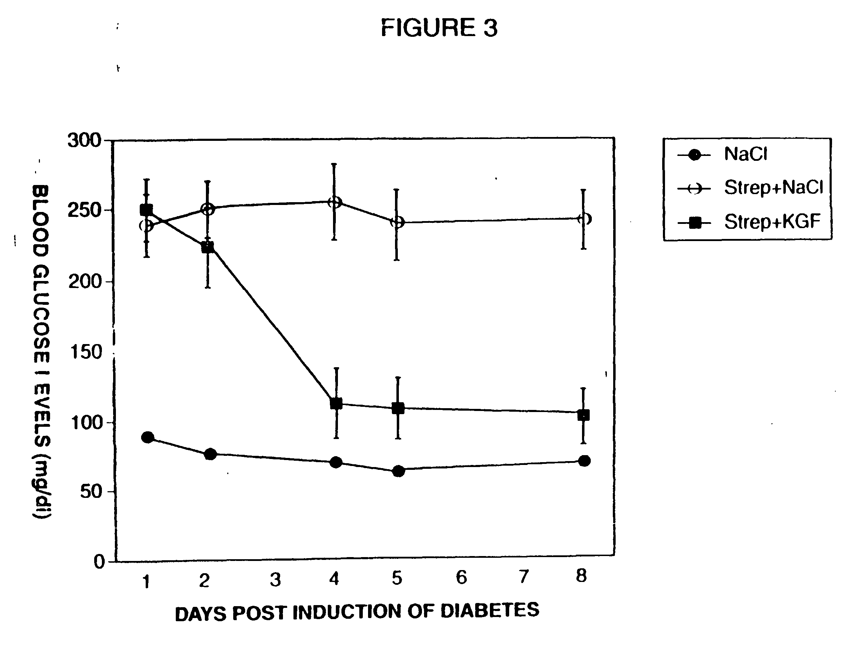

Figure 3 depicts daily non-fasting blood glucose levels in mg/dl in the same rat model of diabetes over an eight day period following diabetes induction. Some animals were treated with a daily subcutaneous dose (3 mg/kg) of KGF beginning one day after disease induction ("Strep + KGF"), while others were pre- and post-treated with sodium chloride solution as a control ("Strep + NaCl"). A non-diabetic group of animals treated with sodium chloride ("NaCl") again served as an additional control. There were six rats per group.

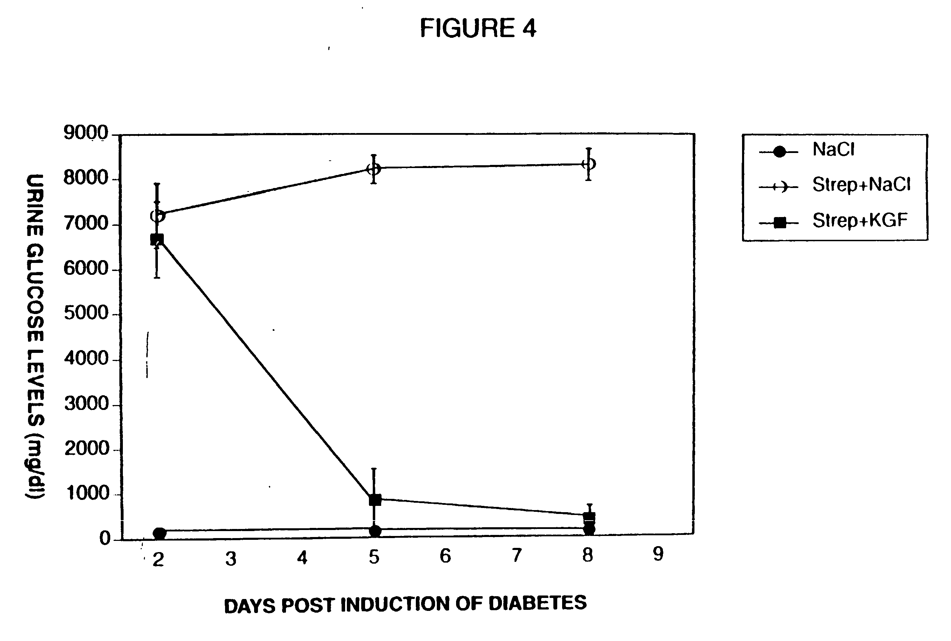

Figure 4 depicts fasting urine glucose levels in mg/dl for the same rat model over a six day period after diabetes induction, in this case beginning on the second day after the induction of disease. KGF treatment was started one day after the induction of diabetes. Graph symbols designate the same three test groups as in Figure 3. There were six rats per group.

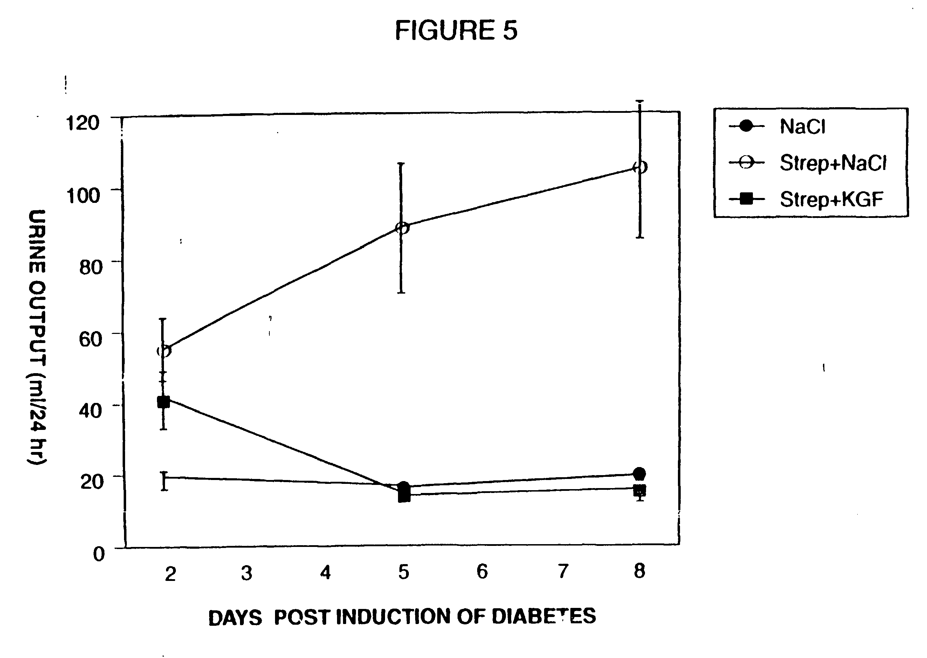

Figure 5 shows the urine output, as milliliters per twenty four hour period, from the same test groups as in Figs. 3 and 4, measured on days 2, 5, 20 and 8 following induction of diabetes. Graph symbols are the same as in Figures 3 and 4. There were six rats per group.

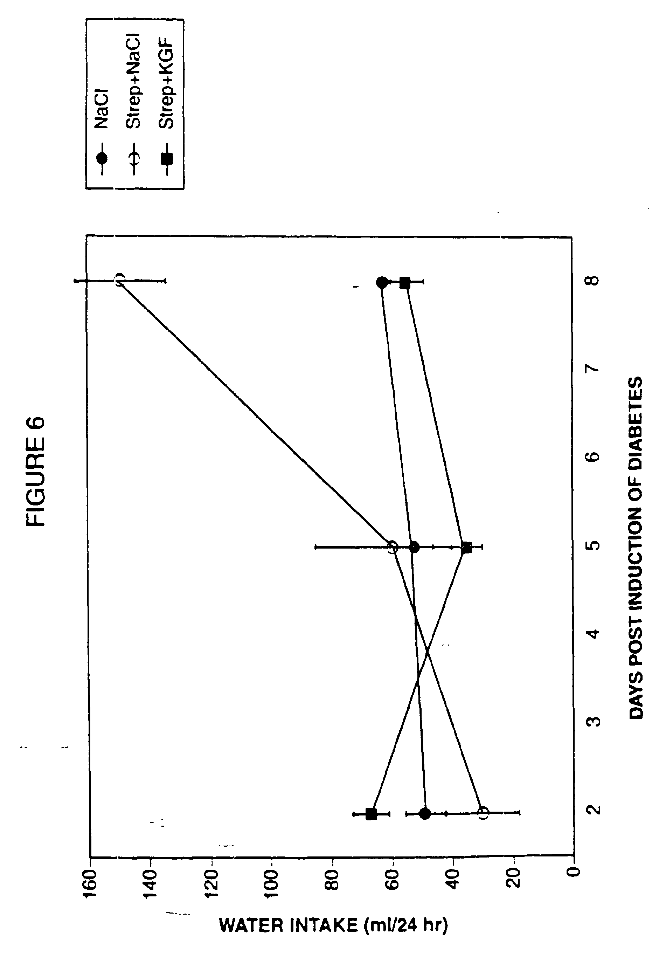

Figure 6 shows the average water intake for each group of rats in the experiment. Rats were given water ad *libitum* and intake was measured as the volume imbibed in milliliters in twenty four hours. There were six rats per group.

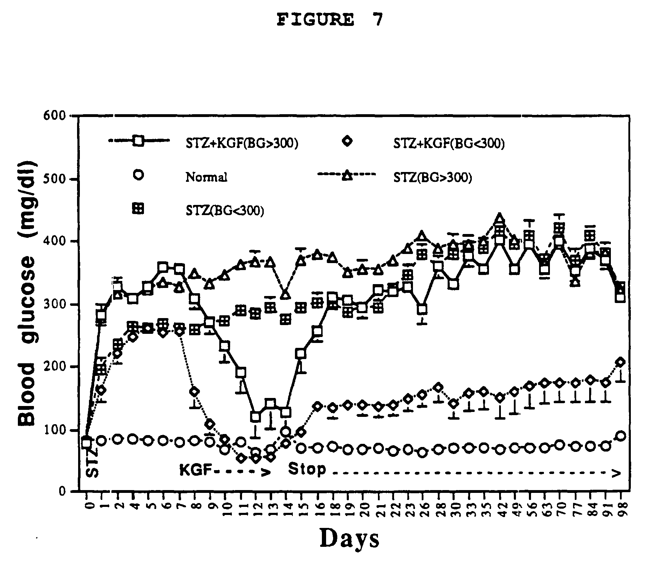

Figure 7 shows the effect of a KGF analog on streptozotocin-induced diabetes in Sprague-Dawley rats.

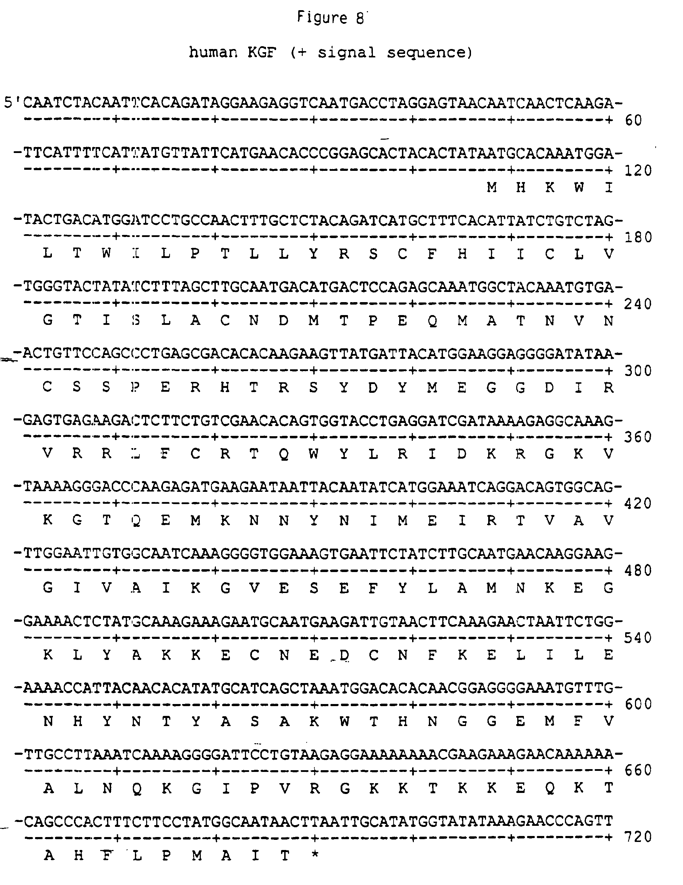

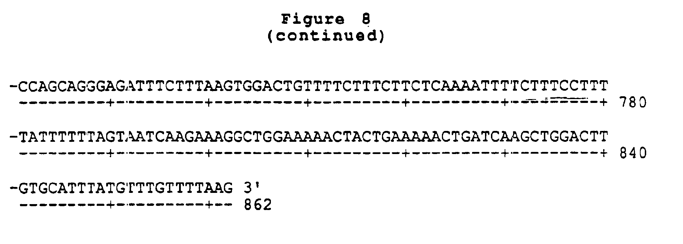

Figure 8 shows the nucleotide (SEQ ID NO:1) and amino acid (SEQ ID NO:2) sequences of native KGF (the nucleotides encoding the mature form of native KGF is depicted by bases 201 to 684 of SEQ ID NO:1 and the mature form of KGF is depicted by amino acid residues 32 to 194 of SEQ ID NO:2).

### DETAILED DESCRIPTION OF THE INVENTION

The present invention is directed to the use of native KGF or a mutein thereof differeing by the deletion, substitution and/or insertion of at least one amino acid from native KGF and having substantially the capability of native KGF to stimulate non-fibroblast epithelial cell proliferation for preparing a medicament for the treatment of diabeters mellitus.

The use of the invention can be practiced using any form of keratinocyte growth factor as defined above having some or all of the biological properties of the naturally-occurring polypeptide as defined above. Such forms include those which are isolated and purified from biological fluids, cells and tissues, or which are derived by chemical synthesis or by recombinant means through expression in heterologous host cells that have been transformed with the encoding DNA or RNA. A recombinant process for production of keratinocyte growth factor is described in the previously mentioned WO 90/08771. Other procedures known to those skilled in the art can be adapted for the same purpose.

By way of illustration, the nucleotide sequence coding for KGF protein, or portion thereof, can be inserted into an appropriate expression vector, i.e., a vector which contains the necessary elements for the transcription and translation of the inserted protein coding sequence. The necessary transcriptional and translation signals can also be supplied by the native KGF gene and/or its flanking regions. A variety of host-vector systems may be utilized to express the protein coding sequence. These include but are not limited to mammalian cell systems infected with virus (e.g., vaccinia virus, adenovirus, etc.); insect cell systems infected with virus (e.g., baculovirus) microorganisms such as yeast containing yeast vectors, or bacteria transformed with bacteriophage DNA, plasmid DNA, or cosmid DNA. The expression elements of these vectors vary in their strengths and specificities. Depending on the host-vector system utilized, any one of a number of suitable transcription and translation elements may be used.

Any of the methods previously described for the insertion of nucleotide fragments into a vector may be used to construct expression vectors containing a chimeric gene consisting of appropriate transcriptional/translational control signals and the protein coding sequences. These methods may include in vitro recombinant DNA and synthetic techniques and *in vivo* recombinations (genetic recombination). Expression of a nucleic acid sequence encoding KGF protein or peptide fragment may be regulated by a second nucleic acid sequence so that KGF protein or peptide is expressed in a host transformed with the recombinant DNA molecule. For example, expression of KGF may be controlled by any promoter/enhancer element known in the art. Promoters which may be used to control KGF expression include, but are not limited to, the SV40 early promoter region, the promoter contained in the 3' long terminal repeat of Rous sarcoma virus, the herpes thymidine kinase promoter, the regulatory sequences of the metallothionein gene, prokaryotic expression vectors such as the β-lactamase promoter, or the tac promoter, plant expression vectors comprising the nopaline synthetase promoter region Herrera-Estrella et *al.*, or the cauliflower mosaic virus 35S RNA promoter, and the promoter for the photosynthetic enzyme ribulose biphosphate carboxylase, promoter elements from yeast or other fungi such as the Gal 4 promoter, the ADC (alcohol dehydrogenase) promoter, PGK (phosphoglycerol kinase) promoter, alkaline phophatase promoter, and the following animal transcriptional control region, which exhibit tissue specificity and have been utilized in transgenic animals: elastase I gene control region which is active in pancreatic acinar cells, insulin gene control region which is active in pancreatic beta cells, immunoglobulin gene control region which is active in lymphoid cells Grosschedl et *al.*, mouse mammary tumor virus control region which is active in testicular, breast, lymphoid and mast cells Leder et *al.,* albumin gene control region which is active in liver, alpha fetoprotein gene control region which is active in liver, alpha 1-antitrypsin gene control region which is active in the liver, beta-globin gene control region which is active in myeloid cells, myelin basic protein gene control region which is active in oligodendrocyte cells in the brain, myosin light chain-2 gene control region which is active in skeletal muscle, and gonadotropic releasing hormone gene control region which is active in the hypothalamus.

Expression vectors containing KGF gene inserts can be identified by DNA-DNA hybridization, presence or absence of "marker" gene functions, and expression of inserted sequences, as will be evident and are familiar to those skilled in the art.

Several methods known in the art may be used to propagate the KGF gene. Once a suitable host system and growth conditions are established, recombinant expression vectors can be propagated and prepared in quantity.

In addition, a host cell strain may be chosen which modulates the expression of the inserted sequences, or modifies and processes the gene product in the specific fashion desired. Expression from certain promoters can be elevated in the presence of certain inducers. Thus, expression of the genetically engineered KGF protein may be controlled. Furthermore, different host cells have characteristic and specific mechanisms for the translational and post-translational processing and modification (for example, glycosylation, gamma carboxylation of glutamic acid residues, proteolytic cleavage) of proteins. Appropriate cell lines or host systems can be chosen to ensure the desired modification and processing of the foreign protein expressed.

It should be understood that the terms "keratinocyte growth factor" and "KGF" as employed in this description are intended to include, and to mean interchangeably unless otherwise indicated, native KGF and KGF analog proteins (or "muteins") characterized by a peptide sequence substantially the same as the peptide sequence of native KGF and by retaining some or all of the biological activity of native KGF, particularly non-fibroblast epithelial cell proliferation (e.g., exhibiting at least about 500-fold greater stimulation of BALB/MK keratinocyte cells than that of NIH/3T3 fibroblast cells, and at least about 50-fold greater stimulation of BALB/MK keratinocyte cells than for BS/589 epithelial cells or for CC1208 epithelial cells, as determined by H-thymidine incorporation). By "characterized by a peptide sequence substantially the same as the peptide sequence of native KGF" is meant a peptide sequence which is encoded by a DNA sequence capable of hybridizing of nucleotides 201 to 684 of SEQ ID NO:1, preferably under stringent hybridization conditions.

The determination of a corresponding amino acid position between two amino acid sequences may be determined by aligning the two sequences to maximize matches of residues including shifting the amino and/or carboxyl terminus, introducing gaps as required and/or deleting residues present as inserts in the candidate. Database searches, sequence analysis and manipulations may be performed using one of the well-known and routinely used sequence homology/identity scanning algorithm programs (e.g., Pearson and Lipman (1988), *Proc. Natl. Acad. Sci. U.S.A.,* 85:2444-2448; Altschul *et al.* (1990), *J. Mol. Biol.,* 215:403-410; Lipman and Pearson (1985), *Science,* 222:1435 or Devereux *et al.* (1984), *Nuc. Acids Res.,* 12:387-395).

Stringent conditions, in the hybridization context, will be stringent combined conditions of salt, temperature, organic solvents and other parameters typically controlled in hybridization reactions. Exemplary stringent hybridization conditions are hybridization in 4 X SSC at 62-67° C., followed by washing in 0.1 X SSC at 62-67° C. for approximately an hour. Alternatively, exemplary stringent hybridization conditions are hybridization in 45-55% formamide, 4 X SSC at 40-45°C. [See, T. Maniatis et. al., *Molecular Cloning* (A Laboratory Manual); Cold Spring Harbor Laboratory (1982), pages 387 to 389].

Thus, the proteins include allelic variations, or deletion(s), substitution(s) or insertion(s) of amino acids., including fragments, chimeric or hybrid molecules of native KGF. One example of KGF includes proteins having residues corresponding to Cys¹ and Cys¹⁵ as referred to in claim 5 replaced or deleted, with the resultant molecule having improved stability as compared with the parent molecule. Another example of KGF includes charge-change polypeptides wherein one or more of amino acid residues 41-154 of native KGF (preferably residues Arg⁴¹, Gln⁴³, Lys⁵⁵, Lys⁹⁵, Lys¹²⁸, Asn¹³⁷, Gln¹³⁸, Lys¹³⁹, Arg¹⁴⁴, Lys¹⁴⁷, Gln¹⁵², Lys¹⁵³ or Thr¹⁵⁴) are deleted or substituted with a neutral residue or negatively charged residue selected to effect a protein with a reduced positive charge. A still further example of KGF includes proteins generated by substituting at least one amino acid having a higher loop-forming potential for at least one amino acid within a loop-forming region of Asn¹¹⁵-His¹¹⁶⁻Tyr¹¹⁷-Asn¹¹⁸-Thr¹¹⁹ of native KGF. A still yet further example includes proteins having one or more amino acid substitutions, deletions or additions within a region of 123-133 (amino acids 154-164 of SEQ ID NO:2) of native KGF; these proteins ; may have agonistic or antagonistic activity.

Specifically disclosed proteins include the following KGF molecules (referred to by the residue found at that position in the mature protein (minus signal sequence) set forth in SEQ ID NO:2, followed by that amino acid position in parentheses and then either the substituted residue or "-" to designate a deletion): C(1,15)S, ΔN15-ΔN24, ΔN3/C(15)S, ΔN3/C(15)-, ΔN8/C(15)S, ΔN8/C(15)-, C(1,15)S/R(144)E, C(1,15)S/R(144)Q, ΔN23/R(144)Q, C(1,15,40)S, C(1,15,102)S, C(1,15,102,106)S, ΔN23/N(137)E, ΔN23/K(139)E, ΔN23/K(139)Q, ΔN23/R(144)A, ΔN23/R(144)E, ΔN23/R(144)L, ΔN23/K(147)E, ΔN23/K(147)Q, ΔN23/K(153)E, ΔN23/K(153)Q, ΔN23/Q(152)E/K(153)E; R(144)Q and H(116)G.

For practical application in therapeutic treatment, KGF can be formulated into appropriate pharmaceutical compositions for administration by any conventional means, including but not limited to parenteral delivery, such as subcutaneous, intravenous or intramuscular injection. These standard formulations would most likely include suitable buffer salts, preservatives and stabilizing agents for a liquid formulation or suitable buffer salts, stabilizing agents, preservatives and bulking agents typical for a lyophilized formulation. It is also possible that KGF could be administered in a slow release form, by intradermal, subcutaneous, or intra-abdominal depot. These slow release forms of KGF can be formulated using standard methods within the skill of those knowledgeable in the art. The most practical administration regimens for KGF can be utilized by the patient at home using subcutaneous injection or intradermal delivery, or by the physician using a long-term slow release formulation implanted subcutaneously or in the peritoneal cavity.

The dosing regimen for KGF can be determined empirically by the skilled practitioner. In general, it is anticipated that KGF will be effective in amounts from about 0.001 to about 10 milligrams per kilogram of body weight (of the patient) per day, and preferably from about 0.05 to about 5 mg/kg/day. The therapeutic regimen can include single or repeated injections, or a slow continuously released low dose of KGF, depending on the type and severity of the disease in each patient. The therapeutic course of treatment with KGF must produce enough pancreatic beta cell function in order to normalize blood glucose levels during varying metabolic demands, yet avoid frequent or profound hypoglycemia. The aim is to replenish the islet cell function of patients with diagnosed Type I diabetes to avoid the necessity of constant exogenous insulin requirements. Patients with newly diagnosed Type I diabetes, in whom some islet cell function remains, would be candidates for KGF therapy. KGF could be used to maintain the islet function of such patients so as to ameliorate, delay, or circumvent permanent manifestation of disease. Type I diabetes is believed to be an autoimmune disease and immunosuppressant therapy is used for its treatment. KGF therapy in accordance with this invention can be used in conjunction or combination with immunosuppressants for treatment of the disease, including as an adjunct in the setting of islet cell transplantation. The invention is further illustrated with reference to the following application.

### Materials

The test materials used in the following *in vivo* studies were, as specifically indicated KGF of native (naturally occurring) sequence, a KGF analog in which the cysteine residues at positions 1 and 15 of the native amino acid sequence had been replaced with serine using standard techniques of site directed mutagenesis (i.e., C(1,15)S) and a KGF analog having a deletion of the first 23 amino acids of the N-terminus of native KGF using standard techniques (i.e., ΔN23). All proteins were produced by recombinant expression in *E. coli* and purified to homogeneity, and they each contained a methionine residue (Met⁻¹) at the N-terminus. Each protein was administered as a subcutaneous formulation. Previous experiments demonstrated that these proteins had comparable activities in adult rats when administered systemically. The native sequence KGF and the analogs each had comparable activities in the diabetic and non-diabetic rats used in the following studies.

### In Vivo Model of Diabetes

Chemically-induced diabetes mellitus models in various animal species have been classically used to study the disease and its treatment. Streptozotocin induces diabetes in the mouse, rat, hamster, dog, and monkey although studies in rats and mice are utilized most. Junod *et al., Proc. Soc. Exp. Pio. Med.* **126:**210-205 (1967); Rerup, *Pharm. Rev.* **22:**485-518 (1970); Rossini *et al.,* P.N.A.S. **74:**2485-2489 (1977); and *Ar'Rajab and Ahren, Pancreas* **8**:50-57 (1993). In rats, doses of streptozotocin from 45 to 70 mg/kg as a single intravenous dose induce stable disease. Doses below 45 mg/kg induce a transient disease state which is reversible. Within one day of streptozotocin injection, the hyperglycemic state is induced. Blood insulin levels remain essentially unchanged compared with normal rats; however, the total content of insulin and C-peptide in the pancreas is severely decreased. Rats manifest the classic signs and symptoms of diabetes in humans: increased blood glucose levels (hyperglycemia), glucose in the urine (glucosuria), increased thirst (polydipsia), increased urination (polyuria), increased appetite (hyperphagia).

The studies described in this disclosure were carried out with the streptozotocin-induced diabetes model in Sprague-Dawley rats. Male rats weighing 200 260 grams at study initiation were used. Diabetes was induced by a single intravenous injection of streptozotocin at 50 mg of streptozotocin in sodium citrate buffer per kg of body weight. Non-diabetic control rats received a single intravenous injection of sodium citrate buffer for control purposes. KGF was administered daily as a subcutaneous injection. The KGF dose was 3 or 5 mg/kg/day, depending upon the experiment. In the first experiment, KGF therapy was initiated two days before diabetes, was induced and continued after the induction of diabetes for a total of eight injections. In the second and third experiments, KGF therapy administered subcutaneously was initiated one day after the induction of diabetes with streptozotocin. In the fourth experiment, a 7 day course of KGF therapy was initiated 7 days after streptozotocin treatment and the animals were then followed for an additional 12 weeks. In all experiments, except for the fourth experiment, blood glucose levels, urine glucose levels and urine volume were used as end points for analysis. Additionally, water intake, urine C-peptide levels, or total pancreatic insulin and C-peptide content were measured in some experiments. In the fourth experiment, the only assessed endpoint was blood glucose.

Because a large fraction of insulin is removed from the circulation by the liver, measurement of peripheral insulin concentrations reflect post-hepatic metabolism events rather than insulin secretion from the pancreas. Therefore, measurements of C-peptide are often made and used as a peripheral marker of insulin secretion. C-peptide is produced from the processing of pro-insulin to insulin. Insulin and C-peptide are secreted from the beta cells in equimolar amounts, and only a small amount of C-peptide is extracted by the liver.

### In Vivo Administration of KGF

First Study: Using the diabetes model described, the effectiveness of KGF to treat diabetes was first evaluated using the following four groups of test rats:
1. Control (non-diabetic) rats pre- and post-treated with subcutaneously administered sodium chloride solution, no streptozotocin;
2. Rats made diabetic with intravenously administered 50 mg/kg of streptozotocin, pre- and post-treated with subcutaneously administered sodium chloride solution;
3. Rats made diabetic with intravenously administered 50 mg/kg streptozotocin, pre- and post-treated with subcutaneously administered native KGF; and
4. Control rats treated with subcutaneously administered native KGF, no streptozotocin.

Rats treated in all four groups were administered with either native KGF at a dose of 5 mg/kg per day or an equal volume of sodium chloride solution over a period of seven days. Two days after the commencement of KGF or sodium chloride administration, the rats in groups 2 and 3 were given a single dose of 55 mg/kg of streptozotocin, administered intravenously. This dose is known to cause moderate diabetes in rats. All rats were monitored for non-fasting blood glucose level, body weight, fasting urine glucose level-and urine output. Seven days after administration of streptozotocin to groups 2 and 3 (i.e., nine days after commencement of the study) the rats in all of the groups were fasted overnight, sacrificed, then necropsied. In each case the pancreas was preserved in zinc formalin, embedded, then processed for routine histopathology.

The non-fasting blood glucose level on the fifth day after administration of streptozotocin was significantly elevated in the diabetic control rats (group 2) in comparison with the non-diabetic control rats (group 1), as seen in Figure 1. Diabetic rats which had been pretreated with KGF before streptozotocin administration and post-treated with KGF (group 3) had a significantly lower non-fasting blood glucose level than non-KGF treated diabetic controls (group 2), but still elevated relative to the non-diabetic control (group 1); see Figure 1. The fasting urine glucose level and urine volume of the group 2 diabetic control rats were significantly elevated on the seventh day of the study (i.e., five days after injection with streptozotocin), as seen in Figure 2. This condition is due to the destruction of the insulin-producing beta-cells in the pancreatic islets and the severe dysregulation of glucose metabolism which results in excretion of glucose in the urine. In contrast to this, the diabetic rats of (group 3, which were pre- and post-treated with KGF, showed significantly less elevation in fasting urine glucose than the diabetic control (group 2). The urine output for the KGF-treated group was also significantly less than for the diabetic control group; see Figure 2.

These results are consistent with the induction of a moderate state of diabetes in the rat using streptozotocin as the inducing agent. Those diabetic rats which were treated with KGF prior to diabetes induction, and for which KGF was also continued after the induction, showed symptoms indicative of a milder form of diabetes. Thus, it can be concluded that the KGF therapy either partially prevented induction of the disease or restored insulin-producing islet cells after streptozotocin-induced beta cell destruction. In order to distinguish between these possibilities, KGF therapy beginning after disease induction was next studied.

Second Study: Using the same diabetes model previously described, the effectiveness of KGF to treat diabetes was further evaluated utilizing the following three groups of test rats:
1. Control rats treated with subcutaneously administered sodium chloride solution, no streptozotocin;
2. Rats made diabetic with intravenously administered streptozotocin, and post-treated with subcutaneously administered sodium chloride solution; and
3. Rats made diabetic with intravenously administered streptozotocin, then post-treated with subcutaneously administered C(1,15)S.

Test rats were administered at a dose of 3 mg/kg per day or sodium chloride solution over a period of thirteen days beginning one day after diabetes induction. The blood glucose level, urine glucose level, volume of urine output and water imbibed each under fasting and non-fasting conditions were monitored throughout the test period. The rats became diabetic in groups 2 and 3 within a day of administration of streptozotocin (Figure 3). KGF therapy began in group 3 at twenty four hours after streptozotocin and was continued daily thereafter. Non-fasting blood glucose was measured on days 1,2,4,5 and 8. As Figure 3 demonstrates, KGF therapy in group 3 was able to decrease the circulating blood glucose level to near that of control rats (group 1) by day 4, and this continued through day 8. The non-fasting urinary excretion of glucose was also measured on days 2,5 and 8. Figure 4 demonstrates that KGF therapy decreased urinary glucose levels over 8-fold. Similarly, urine output in the KGF-treated diabetic rats was also normalized when measured on day 5 and day 8 (Figure 5). Water intake, in milliliters per twenty-four hour period, did not increase in the KGF-treated diabetic rats as it did in the diabetic rats receiving sodium chloride solution as a control (Figure 6). The rats were fasted overnight on day 8 and fasting blood glucose levels on day 9 were not different between these three groups. Fasting water intake and urine output were significantly less in the KGF-treated diabetic rats when compared to diabetic rats on day 9, which is further indicative of amelioration of the disease condition. -

Third Study: The third study was a repeat of the second study and confirmed the data presented in Figures 3-6. Additionally, in this experiment, when the rats were necropsied the entire pancreas was removed from each rat and the insulin and C-peptide was extracted and quantitated. Table 1, below, shows the average amount of insulin or C-peptide extractable from the pancreas in each of the three groups. KGF therapy was able to increase the total content of insulin and C-peptide in the pancreas of diabetic rats when compared to diabetic rats treated with sodium chloride solution.

**TABLE 1**

| | Total Pancreatic Content of: | |
|---|---|---|
| Group¹ | Insulin (µg) | C-Peptide (µmole) |
| Control | 83.7 ± 6.7² | 3.5 ± 0.1 |
| Diabetic plus NaCl therapy | 6.4 ± 3.3 | 0.4 ± 0.1 |
| Diabetic plus KGF therapy | 18.9 ± 7.4 | 1.0 ± 0.3 |

| | | |
|---|---|---|
| ¹ n=3-4 rats per group ² Average ± S,E. | | |

The fourth study investigated the effect of KGF on streptozotocin-induced diabetes in Sprague-Dawley rats. On day 0, groups of rats were exposed to either 45 or 50 mg/kg streptozotocin (STZ). Following these treatments, non-fasting blood glucose levels were monitored daily to assess the severity of the islet injury. On day 5, the STZ-treated animals were placed into one of two groups (20/group) depending on the magnitude of hyperglycemia. The dividing point was set at a blood glucose level of 300 mg/dl. A group of non STZ-treated animals served as controls. On day 7, 10 animals from each hyperglycemic group were given ΔN23 (3 mg/kg/day) or PBS by subcutaneous injection for 7 days. Blood glucose levels were then monitored daily, every other day, or weekly and are set forth in Figure 7. Note that STZ-treated animals from both groups receiving KGF had significant declines in blood glucose during the KGF dosing period. Importantly, the mean blood glucose drop experienced by the STZ-treated animals from the <300 mg/dl starting blood glucose group stabilized at about 150 mg/dl whereas the blood glucose drop seen in the >300 mg/dl starting blood glucose group was only transient. Note that the day scale is non-linear.

## Claims

1. Use of native KGF or a mutein thereof differing by the deletion, substitution and/or insertion of at least one amino acid from native KGF and having substantially the capability of native KGF to stimulate non-fibroblast epithelial cell proliferation for preparing a medicament for the treatment of diabetes mellitus.

2. The use according to claim 1, wherein the native KGF or the mutein thereof is a recombinantly produced keratinocyte growth factor.

3. The use according to any one of claims 1 to 2, wherein the native KGF or the mutein thereof is encoded by a nucleic acid sequence comprising a sequence selected from the group consisting of:
(i) the DNA sequence as depicted by bases 201 to 684 of SEQ ID NO:1 or a coding portion thereof;
(ii) a nucleic acid sequence encoding the KGF polypeptide of SEQ ID NO:2: and
(iii) a sequence which hybridizes to a complementary sequence of (i) or (ii).

4. The use according to any one of claims 1 to 2, wherein the native KGF or the mutein thereof comprises an amino acid sequence of mature keratinocyte growth factor (as depicted by amino acid residues 32-194 of SEQ ID NO.2) or a fragment thereof, or an analog thereof.

5. The use according to claim 4, wherein the native KGF or the mutein thereof is selected from the following: C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8/C(15)S, ΔN8/C(15)-, C(1,15)S/R(144)E, C(1,15)S/R(144)Q, ΔN23/R(144)Q, C(1,15,40)S,C(1,15,102)S, C(1,15,102,106)S, ΔN23(137)E, ΔN23/K(139)E, ΔN23/K(139)Q, ΔN23/R(144)A, ΔN23R(144)E, ΔN23/R(144)L, ΔN23/K(147)E, ΔN23/K(147)Q, ΔN23/K(153)E, ΔN23/K(153)Q, ΔN23/Q(152)E/K(153)E, R(144)Q or H(116)G, with each molecule designated by reference to the residue found at that position in mature keratinocyte growth factor, as depicted by amino acid residues 32-194 of SEQ ID NO:2.

6. The use according to any one of claims 2 to 5, wherein the native KGF or the mutein thereof is recombinantly produced in bacterial cells.

7. The use according to any one of claims 4 to 6, wherein said native KGF or the mutein thereof has an amino-terminal methionine.

8. The use according to claim 7, wherein the native KGF or the mutein thereof is produced in *E. coli.*

9. The use according to any one of claims 1 to 8, wherein the native KGF or the mutein thereof is formulated with a pharmaceutically acceptable carrier.

10. The use according to claim 9, wherein the pharmaceutically acceptable carrier is a long-term, slow release formulation.

11. The use according to any one of claims 1 to 10, wherein the native KGF or the mutein thereof is to be administered by parenteral injection, such as subcutaneous, intravenous or intramuscular injection.

12. The use according to any one of claims 1 to 11, wherein the native KGF or the mutein thereof is to be administered in an amount from 0.001 milligrams to 10 milligrams per kilogram of body weight per day.

13. The use according to claim 12, wherein the amount of native KGF or the mutein thereof is from 0.05 milligrams to 5 milligrams per kilogram per day.

14. The use according to any one of claims 1 to 13, wherein the patient is a human.

## Patentansprüche

1. Verwendung von nativem KGF oder einem Mutein davon, das sich durch die Deletion, Substitution und/oder Insertion von mindestens einer Aminosäure von nativem KGF unterscheidet und im Wesentlichen die Fähigkeit von nativem KGF hat, die Proliferation von Epithelzellen, bei denen es sich nicht um Fibroblasten handelt, zu stimulieren, zum Herstellen eines Medikaments zur Behandlung von Diabetes mellitus.

2. Verwendung gemäß Anspruch 1, wobei es sich bei dem nativen KGF oder dem Mutein davon um einen rekombinant hergestellten Keratinozytenwachstumsfaktor handelt.

3. Verwendung gemäß einem der Ansprüche 1 bis 2, wobei das native KGF oder das Mutein davon durch eine Nukleinsäuresequenz kodiert wird, die eine Sequenz umfasst, ausgewählt aus der Gruppe bestehend aus:
(i) der DNA-Sequenz, die durch die Basen 201 bis 684 von SEQ ID NO:1 oder ein kodierendes Teilstück davon dargestellt wird;
(ii) einer Nukleinsäuresequenz, die das KGF-Polypeptid von SEQ ID NO:2 kodiert; und
(iii) einer Sequenz, die an eine komplementäre Sequenz von (i) oder (ii) hybridisiert.

4. Verwendung gemäß einem der Ansprüche 1 bis 2, wobei das native KGF oder das Mutein davon eine Aminosäuresequenz von reifem Keratinozytenwachstumsfaktor (wie durch die Aminosäurereste 32-194 von SEQ ID NO:2 dargestellt) oder ein Fragment davon oder ein Analogon davon umfasst.

5. Verwendung gemäß Anspruch 4, wobei das native KGF oder das Mutein davon aus den Folgenden ausgewählt ist: C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8/C(15)S, ΔN8/C(15)-, C(1,15)S/R(144)E, C(1,15)S/R(144)Q, ΔN23/R(144)Q, C(1,15,40)S, C(1,15,102)S, C(1,15,102,106)S, ΔN23(137)E, ΔN23/K(139)E, ΔN23/K(139)Q, ΔN23/R(144)A, ΔN23R(144)E, ΔN23/R(144)L, ΔN23/K(147)E, ΔN23/K(147)Q, ΔN23/K(153)E, ΔN23/K(153)Q, ΔN23/Q(152)E/K(153)E, R(144)Q oder H(116)G, wobei jedes Molekül durch Bezug auf den Rest bezeichnet wird, der in reifem Keratinozytenwachstumsfaktor an dieser Position gefunden wird, wie durch die Aminosäurereste 32 - 194 von SEQ ID NO:2 dargestellt wird.

6. Verwendung gemäß einem der Ansprüche 2 bis 5, wobei das native KGF oder das Mutein davon in Bakterienzellen rekombinant hergestellt wird.

7. Verwendung gemäß einem der Ansprüche 4 bis 6, wobei das native KGF oder das Mutein davon ein aminoterminales Methionin hat.

8. Verwendung gemäß Anspruch 7, wobei das native KGF oder das Mutein davon in *E. coli* hergestellt wird.

9. Verwendung gemäß einem der Ansprüche 1 bis 8, wobei das native KGF oder das Mutein davon mit einem pharmazeutisch verträglichen Träger formuliert wird.

10. Verwendung gemäß Anspruch 9, wobei der pharmazeutisch verträgliche Träger eine Langzeitformulierung mit langsamer Freisetzung ist.

11. Verwendung gemäß einem der Ansprüche 1 bis 10, wobei das native KGF oder das Mutein davon durch parenterale Injektion, wie zum Beispiel subkutane, intravenöse oder intramuskuläre Injektion, zu verabreichen ist.

12. Verwendung gemäß einem der Ansprüche 1 bis 11, wobei das native KGF oder das Mutein davon in einer Menge von 0,001 Milligramm bis 10 Milligramm pro Kilogramm Körpergewicht pro Tag zu verabreichen ist.

13. Verwendung gemäß Anspruch 12, wobei die Menge an nativem KGF oder dem Mutein davon von 0,05 Milligramm bis 5 Milligramm pro Kilogramm pro Tag beträgt.

14. Verwendung gemäß einem der Ansprüche 1 bis 13, wobei es sich bei dem Patienten um einen Menschen handelt.

## Revendications

1. Utilisation de KGF natif ou d'une mutéine de celui-ci différant par la délétion, la substitution et/ou l'insertion d'au moins un acide aminé par rapport au KGF natif, et ayant substantiellement la capacité du KGF natif à stimuler la prolifération des cellules épithéliales non fibroblastes, pour la préparation d'un médicament pour le traitement du diabète sucré.

2. Utilisation selon la revendication 1, dans laquelle le KGF natif ou la mutéine de celui-ci est un facteur de croissance des kératinocytes produit de façon recombinante.

3. Utilisation selon l'une quelconque des revendications 1 à 2, dans laquelle le KGF natif ou la mutéine de celui-ci est codé par une séquence d'acide nucléique comprenant une séquence choisie dans le groupe constitué de :
(i) la séquence d'ADN telle que décrite par les bases 201 à 684 de la SEQ ID n° : 1 ou une partie codante de celle-ci ;
(ii) une séquence d'acide nucléique codant pour le polypeptide du KGF de la SEQ ID n° : 2 ; et
(iii) une séquence qui s'hybride à une séquence complémentaire de (i) ou de (ii).

4. Utilisation selon l'une quelconque des revendications 1 à 2, dans laquelle le KGF natif ou la mutéine de celui-ci comprend une séquence d'acides aminés du facteur de croissance des kératinocytes mature (comme décrit par les résidus d'acides aminés 32 à 194 de la SEQ ID n° : 2) ou un fragment de celui-ci ou un analogue de celui-ci.

5. Utilisation selon la revendication 4, dans laquelle le KGF natif ou la mutéine de celui-ci est choisi parmi les suivants : C(1,15)S, ΔN15, ΔN16, ΔN17, ΔN18, ΔN19, ΔN20, ΔN21, ΔN22, ΔN23, ΔN24, ΔN3/C(15)S, ΔN3/C(15)-, ΔN8/C(15)S, ΔN8/ C(15)-, C(1,15)S/R(144)E, C(1,15)S/R(144)Q, ΔN23/R(144)Q, C(1,15,40)S, C(1,15,102)S, C(1,15, 102,106)S, ΔN23(137)E, ΔN23/K(139)E, ΔN23/ K(139)Q, ΔN23/R(144)A, ΔN23R(144)E, ΔN23/ R(144)L, ΔN23/K(147)E, ΔN23/K(147)Q, ΔN23/ K(153)E, ΔN23/K(153)Q, ΔN23/Q(152)E/K(153)E, R(144)Q ou H(116)G, avec chaque molécule désignée par la référence au résidu trouvé à cette position dans le facteur de croissance des kératinocytes mature, comme décrit par les résidus d'acides aminés 32 à 194 de la SEQ ID n° : 2.

6. Utilisation selon l'une quelconque des revendications 2 à 5, dans laquelle le KGF natif ou la mutéine de celui-ci est produit de façon recombinante dans des cellules bactériennes.

7. Utilisation selon l'une quelconque des revendications 4 à 6, dans laquelle ledit KGF natif ou la mutéine de celui-ci a une méthionine amino-terminale.

8. Utilisation selon la revendication 7, dans laquelle le KGF natif ou la mutéine de celui-ci est produit dans *E. coli.*

9. Utilisation selon l'une quelconque des revendications 1 à 8, dans laquelle le KGF natif ou la mutéine de celui-ci est formulé avec un transporteur pharmaceutiquement acceptable.

10. Utilisation selon la revendication 9, dans laquelle le transporteur pharmaceutiquement acceptable est une formulation à libération lente, à long terme.

11. Utilisation selon l'une quelconque des revendications 1 à 10, dans laquelle le KGF natif ou la mutéine de celui-ci doit être administré par injection parentérale, telle qu'une injection souscutanée, intraveineuse ou intramusculaire.

12. Utilisation selon l'une quelconque des revendications 1 à 11, dans laquelle le KGF natif ou la mutéine de celui-ci doit être administré dans une quantité allant de 0,001 milligramme à 10 milligrammes par kilogramme de poids corporel par jour.

13. Utilisation selon la revendication 12, dans laquelle la quantité du KGF natif ou de la mutéine de celui-ci va de 0,05 milligramme à 5 milligrammes par kilogramme par jour.

14. Utilisation selon l'une quelconque des revendications 1 à 13, dans laquelle le patient est un être humain.
